# FASCICULE DE BREVET EUROPEEN

(11) **EP 3 448 834 B1**
(45) Date de publication et mention de la délivrance du brevet: **15.03.2023**
(21) Numéro de dépôt: 17724855.6
(22) Date de dépôt: 24.04.2017
(51) Int. Cl.: C07C 213/08, C07C 213/10

(54) **COMPOSITION D'ACRYLATE DE N,N-DIMETHYL AMINOETHYLE STABILISEE VIS-A-VIS DE LA COLORATION**
HINSICHTLICH ENTFÄRBUNGSEFFEKTEN STABILISIERTE N,N-DIMETHYLAMINOETHYL-ACRYLAT-ZUSAMMENSETZUNG
N,N-DIMETHYLAMINOETHYL ACRYLATE COMPOSITION STABILIZED IN RESPECT OF DISCOLORING EFFECTS

(30) Priorité: 28.04.2016 FR 1653798
(43) Date de publication de la demande: 06.03.2019
(73) Titulaire: Arkema France, 92700 Colombes (FR)
(72) Inventeur: DUBUT, Fanny, 57070 METZ (FR); RIFLADE, Benoit, 33430 BAZAS (FR); MAGET, Sandra, 57490 CARLING (FR)
(74) Mandataire: Arkema Patent
(86) Numéro de dépôt international: PCT/FR2017/050960
(87) Numéro de publication internationale: WO 2017/187067

(56) Documents cités:
- EP-A1- 0 850 916
- EP-A2- 0 960 877
- US-A1- 2004 171 868
- US-B1- 6 376 703

## Description

### Domaine technique

La présente invention est définie par les revendications.

### Art antérieur et problème technique

L'acrylate de N,N-diméthyl aminoéthyle (désigné ci-après par ADAME) répondant à la formule (I) :

H₂C=CH-COOCH₂CH₂N(CH₃)₂ ( I )

est obtenu par réaction de transestérification entre un acrylate d'alkyle léger (dénommé aussi acrylate léger) de formule (II) : CH₂=CH-COOR₁ dans laquelle R₁ représente le radical méthyle ou éthyle, et le N,N-diméthyl aminoéthanol (DMAE), selon le schéma réactionnel suivant :

CH₂=CH-COOR₁ + HO-CH₂CH₂N(CH₃)₂ ⇔ ADAME + R₁OH (1)

La réaction s'effectue en général en présence d'un excès d'acrylate d'alkyle léger et la réaction est déplacée dans le sens de la formation de l'ADAME en distillant l'alcool léger R₁OH sous forme d'un azéotrope acrylate léger / alcool R₁OH. La réaction s'accompagne de réactions secondaires produisant des impuretés dans le milieu réactionnel. Le milieu réactionnel est ensuite soumis à un ensemble de traitements visant à séparer le produit pur.

L'acrylate de N,N-diméthyl aminoéthyle (ADAME) et plus généralement les (méth)acrylates de dialkylaminoalkyle sont des matières premières pour la préparation de sels quaternaires pour obtenir des polymères cationiques, utilisables comme floculants dans le domaine du traitement des eaux, comme agents de traitement du papier ou du textile, ou dans le domaine de l'exploitation minière et de l'industrie du pétrole et du gaz.

Le manque de stabilité au cours du temps sous certaines conditions (de température par exemple) de ces composés, notamment leur jaunissement, pose problème. En effet, la préparation de sels quaternaires à partir de ces matières premières peut conduire à des solutions aqueuses colorées de façon aléatoire, et à la formation de polymères cationiques colorés qui peuvent poser des problèmes de coloration de l'eau lorsqu'ils sont ensuite utilisés dans le traitement des eaux.

Pour résoudre ou au moins masquer ce problème d'instabilité de couleur, l'ADAME nécessite d'être stocké sous certaines conditions de température, en particulier à une température inférieure à 5°C à l'abri de la lumière, compliquant ainsi les installations de stockage ou de transport du produit. En l'absence de ces précautions prises pour le stockage, l'ADAME est utilisé rapidement après sa production pour éviter les problèmes induits par l'évolution possible de sa coloration.

Les études réalisées dans l'art antérieur sur la production de l'ADAME, ont essentiellement été menées dans le but d'obtenir un produit fini de haute pureté.

A titre d'exemple, le document EP 0960 877A2 décrit un processus de traitement d'un milieu réactionnel obtenu par transestérification d'acrylate d'éthyle (AE) et de diméthylaminoéthanol (DMAE) en présence d'un catalyseur à base de titanate d'alkyle. Ce processus est basé sur un équeutage (élimination du catalyseur et des sous-produits lourds), suivi d'un étêtage (élimination des composés légers) et d'une rectification finale, conduisant à un produit final de haute pureté, comportant moins de 100 ppm d'AE et moins de 300 ppm de DMAE.

Dans le procédé décrit dans le document FR 2 811 986, il a été proposé d'utiliser comme catalyseur le titanate de tétraéthyle en solution dans le DMAE pour éviter en outre les impuretés générées par le catalyseur.

D'autres études ont porté sur l'optimisation du procédé de production de l'ADAME, permettant le recyclage de la fraction azéotropique générée lors de la réaction de transestérification, après purification, sur une unité de production d'acrylate d'alkyle (WO 2010/086547), ou sur la valorisation des produits nobles récupérés à partir du traitement thermique des sous-produits lourds formés au cours du procédé (WO 2013/045786).

Le problème de la stabilisation des dérivés (méth)acryliques, en particulier de l'ADAME, du fait de leur faculté à polymériser, est résolu par l'utilisation d'inhibiteurs de polymérisation introduits au cours de leurs procédés de fabrication ou de purification. Quant au problème de l'évolution de la coloration de l'ADAME au cours du temps, il est proposé dans le brevet EP 0850 916, d'ajouter au moins un composé choisi parmi les composés contenant un groupe amido, les esters de l'acide phosphoreux, les esters de l'acide phosphorique et les phosphines, et au moins un composé phénolique substitué. Les exemples montrent que la présence des deux composés est nécessaire, afin de garantir la stabilisation de l'ADAME.

La présente invention a donc pour but de fournir une méthode mieux adaptée au contexte industriel pour stabiliser l'ADAME au niveau de sa coloration, et donc pour garantir sa stabilité vis-à-vis de la coloration durant le stockage.

Les inventeurs ont découvert, de manière surprenante, que l'addition sous certaines conditions du composé 2,6-di-tert-butyl-4-méthyl phénol permettait d'obtenir un ADAME stabilisé vis-à-vis de la coloration, indépendamment des conditions de synthèse ou de purification du produit.

Le 2,6-di-tert-butyl-4-méthyl phénol (dénommé aussi 2,6-di-tert-butyl-p-crésol) est un composé qui fait partie des inhibiteurs de polymérisation utilisables dans les procédés de fabrication et de purification de dérivés (méth)acryliques au même titre que la phénothiazine, l'hydroquinone, l'éther méthylique d'hydroquinone, le tertiobutyl catéchol ou les composé nitroxyls TEMPO.

Par exemple dans le procédé de synthèse d'esters (méth)acryliques décrit dans le brevet US 7,294,240, de l'ordre de 10-20 ppm d'éther méthylique d'hydroquinone ou de 2,6-di-tert-butyl-p-crésol sont introduits au niveau du soutirage latéral de l'ester (méth)acrylique purifié afin de stabiliser le produit recherché. Dans le procédé décrit dans le document US 2004/171868, l'ester (méth)acrylique produit est stabilisé au stockage par l'addition d'un agent stabilisant, choisi de préférence parmi le 2,6-di-tert-butyl-p-crésol et l'éther méthylique d'hydroquinone, à une teneur pouvant aller de 5 à 500 ppm. Cependant, l'effet du 2,6-di-tert-butyl-4-méthyl phénol seul, sur l'inhibition de la coloration de l'ADAME n'est pas décrit dans ces documents et n'a jamais été suggéré dans l'art antérieur.

### Résumé de l'invention

Décrit ici est une composition d'acrylate de N,N-diméthyl aminoéthyle stabilisée vis-à-vis de la coloration, comprenant de 25 à 200 ppm de 2,6-di-tert-butyl-4-méthyl phénol.

Par « stabilisée vis-à-vis de la coloration » on entend que l'évolution de la coloration est au moins deux fois moins rapide en présence de 2,6-di-tert-butyl-4-méthyl phénol qu'en l'absence de 2,6-di-tert-butyl-4-méthyl phénol, lorsque la composition est stockée à une température allant de 0 à 40°C pendant une durée pouvant aller d'au moins 10 jours jusqu'à 30 jours à l'abri de la lumière.

Selon un mode de réalisation, la composition stabilisée présente une coloration inférieure à 100 Apha, de préférence inférieure à 30 Apha, plus préférentiellement inférieure à 20 Apha déterminée par mesure de l'absorbance de la composition à l'aide d'un colorimètre. De préférence, la composition stabilisée présente une coloration inférieure à 20 Apha après stockage à température ambiante (environ 20°C) à l'abri de la lumière pendant une période de 30 jours.

Objet de l'invention est un procédé de fabrication en continu d'une composition d'acrylate de N,N-diméthyl aminoéthyle stabilisée vis-à-vis de la coloration, comprenant une réaction de transestérification entre un acrylate léger choisi parmi l'acrylate de méthyle et l'acrylate d'éthyle, et le diméthylaminoéthanol en présence d'un catalyseur, suivie d'un traitement de purification du mélange réactionnel comprenant une distillation finale de l'acrylate de N,N-diméthyl aminoéthyle purifié, caractérisé en ce que l'on introduit de 25 à 200 ppm de 2,6-di-tert-butyl-4-méthyl phénol à la sortie du flux gazeux distillé d'acrylate de N,N-diméthyl aminoéthyle purifié, avant condensation du produit final.

Également décrit est l'utilisation du 2,6-di-tert-butyl-4-méthyl phénol à une teneur comprise entre 25 et 200 ppm pour stabiliser vis-à-vis de la coloration l'acrylate de N,N-diméthyl aminoéthyle.

De préférence, on utilise l'acrylate d'éthyle comme acrylate léger pour la réaction de transestérification.

Le 2,6-di-tert-butyl-4-méthyl phénol, introduit en faible teneur à la fin du processus de purification de l'ADAME inhibe l'évolution de la coloration de l'ADAME, et permet ainsi une plus longue durée de stockage du produit à des températures plus élevées que la température généralement nécessaire, en particulier permet le stockage à température ambiante.

L'invention est maintenant décrite plus en détail et de façon non limitative dans la description qui suit.

### Exposé détaillé de l'invention

Le 2,6-di-tert-butyl-4-méthyl phénol, (ou 2,6-di-tert-butyl-p-crésol ou hydroxytoluène butylé) (dénommé dans la suite de l'exposé BHT) de numéro CAS 128-37-0 est disponible commercialement sous la forme de poudre blanche cristalline.

Le BHT présent dans de l'ADAME purifié à une teneur allant de 25 à 200 ppm, de préférence de 25 à 100 ppm, plus préférentiellement de 30 à 80 ppm, plus particulièrement de 30 à 50 ppm, conduit à une composition stabilisée vis-à-vis de la coloration, utilisable comme matière première pour préparer des polymères.

Le BHT est introduit tel quel ou sous la forme d'une solution qui peut être préparée dans l'ADAME mais également dans les matières premières utilisées pour obtenir l'ADAME, tel que l'acrylate d'alkyle léger ou l'alcool lourd, en présence d'autres stabilisants, tels que l'EMHQ.

La composition décrite ici peut comprendre en outre au moins un stabilisant pour inhiber la polymérisation de l'ADAME, choisi par exemple parmi l'hydroquinone, l'éther méthylique d'hydroquinone (EMHQ) ou leurs mélanges à une teneur comprise généralement entre 200 ppm et 1000 ppm, de préférence entre 500 et 800 ppm. On utilise généralement l'EMHQ comme inhibiteur de polymérisation de l'ADAME à une teneur de l'ordre de 700 ppm. Selon un mode de réalisation préféré, la composition comprend 800 ppm d'EMHQ.

La composition comprend en outre des impuretés résiduelles en faible teneur.

La composition stabilisée vis-à-vis de la coloration présente une coloration inférieure à 100 Apha, de préférence inférieure à 30 Apha, plus préférentiellement inférieure à 20 Apha. La coloration de la composition est stabilisée, c'est-à-dire que son évolution est au moins deux fois moins rapide du fait de la présence de BHT qu'en l'absence de BHT, lorsque la composition est stockée à une température allant de 0 à 40°C pendant une durée pouvant aller d'au moins 10 jours jusqu'à 30 jours à l'abri de la lumière. La composition peut ainsi être stockée à l'abri de la lumière à température ambiante sans que sa coloration n'évolue rapidement. Avantageusement, la composition présente une coloration inférieure à 20 Apha après stockage à 20°C à l'abri de la lumière durant environ 30 jours.

La composition est utilisée pour préparer des polymères cationiques, utilisables comme floculants dans le domaine du traitement des eaux, comme agents de traitement du papier ou du textile, ou dans le domaine de l'exploitation minière et de l'industrie du pétrole et du gaz.

Avantageusement, le BHT est introduit en continu à la fin du traitement de purification de l'ADAME, en même temps que l'EMHQ, en particulier dans le distillat final du produit purifié qui est ensuite condensé pour récupérer la composition stabilisée d'ADAME purifié.

Le traitement de purification de l'ADAME peut être réalisé de différentes façons, et mettre en oeuvre différentes techniques de séparation et/ou extraction bien connues de l'homme de l'art, en particulier à l'aide de colonnes de distillation, et/ou d'évaporateurs à film et/ou de condenseurs, décanteurs, etc.

Plus particulièrement, le procédé de purification peut comprendre une étape d'équeutage (élimination des sous-produits lourds et du catalyseur) suivie d'une étape d'étêtage (élimination des sous-produits légers), ou inversement peut comprendre une étape d'étêtage suivie d'une étape d'équeutage, éventuellement suivies d'une rectification. Le produit purifié peut être récupéré en soutirage latéral de la colonne de distillation utilisée pour l'étêtage et/ou l'équeutage final, ou en tête de la colonne de rectification finale.

Selon un mode de réalisation particulier, un procédé de fabrication en continu d'une composition d'acrylate de N,N-diméthyl aminoéthyle stabilisée vis-à-vis de la coloration telle que définie selon l'invention, peut comprendre les étapes suivantes :
a) on effectue une réaction de transestérification entre un acrylate léger choisi parmi l'acrylate de méthyle et l'acrylate d'éthyle, et du diméthylaminoéthanol en présence d'un catalyseur de transestérification, et en présence d'au moins un inhibiteur de polymérisation, le mélange azéotropique acrylate léger / alcool léger correspondant étant soutiré en continu pendant la réaction ;
b) on adresse le milieu brut réactionnel à une première colonne de distillation C1 sous pression réduite, dite colonne d'équeutage, le mélange brut réactionnel comprenant l'acrylate de N,N-diméthyl aminoéthyle recherché avec, comme produits légers, le diméthylaminoéthanol et l'acrylate léger n'ayant pas réagi, et, comme produits lourds, le catalyseur, le ou les inhibiteurs de polymérisation ainsi que des sous-produits lourds de réaction, permettant d'obtenir :
   o en tète, un flux composé essentiellement d'acrylate de N,N-diméthyl aminoéthyle et des produits légers, comportant une fraction minoritaire de produits lourds, mais exempt ou sensiblement exempt de catalyseur ; et
   o en pied, un flux composé de produits lourds avec une fraction minoritaire d'acrylate de N,N-diméthyl aminoéthyle et le catalyseur ; puis
c) on adresse le flux de tête de la première colonne de distillation C1 à une seconde colonne de distillation C2 sous pression réduite, dite colonne d'étêtage, permettant d'obtenir :
   o en tête, un flux des produits légers avec une fraction minoritaire d'acrylate de N,N-diméthyl aminoéthyle ; et
   o en pied, l'acrylate de N,N-diméthyl aminoéthyle contenant des traces de produits légers, des sous-produits de réaction lourds et le ou les inhibiteurs de polymérisation ; puis
d) on adresse le flux de pied de la seconde colonne de distillation C2 à une troisième colonne de distillation C3 sous pression réduite, dite colonne de rectification, dans laquelle on introduit en tête de 25 à 200 ppm de 2,6-di-tert-butyl-4-méthyl phénol, permettant d'obtenir :
   o en tête, un flux gazeux d'acrylate de N,N-diméthyl aminoéthyle purifié et stabilisé vis-à-vis de la coloration qui est ensuite condensé ; et
   o en pied, essentiellement le ou les inhibiteurs de polymérisation.

Il est bien entendu que le procédé ci-dessus peut comprendre d'autres étapes préliminaires, intermédiaires ou subséquentes, pour autant qu'elles n'affectent pas négativement l'obtention de l'acrylate de N,N-diméthyl aminoéthyle purifié, ou pourtant qu'elles améliorent la productivité du procédé.

Les conditions opératoires de mise en oeuvre des étapes a) à d) du procédé selon l'invention, notamment les conditions réactionnelles ou les conditions de distillation, sont celles bien connues de l'homme de l'art, et peuvent être celles décrites dans le document EP 0960 877A2.
Les exemples ci-après illustrent la présente invention sans toutefois en limiter la portée.

### EXEMPLES

La coloration de différents échantillons d'ADAME contenant ou non du BHT a été mesurée dans différentes conditions à l'aide d'un colorimètre LANGE LICO 400 par rapport à un étalon de 10 Apha.

### Exemple 1 (selon l'invention)

Un échantillon d'ADAME comprenant 46 ppm de BHT, a été conservé à l'abri de la lumière à 20°C, pendant 30 jours.

La coloration de l'échantillon a été déterminée à t = 0, après 10 jours, après 20 jours et après 30 jours.
t = 0 : 8 Apha ; t = 10 jours : 09 Apha ; t = 20 jours : 12 Apha ; t = 30 jours : 15 Apha

### Exemple 2 (comparatif)

Un échantillon d'ADAME ne comprenant pas de BHT, a été conservé dans les mêmes conditions que celles de l'exemple 1.

La coloration de l'échantillon a été déterminée à t = 0, après 10 jours, après 20 jours et après 30 jours.
t = 0 : 8 Apha ; t = 10 jours : 23 Apha ; t = 20 jours : 45 Apha ; t = 30 jours : 72 Apha

### Exemple 3 (selon l'invention)

Un échantillon d'ADAME comprenant 46 ppm de BHT, a été conservé à l'abri de la lumière à 30°C, pendant 30 jours.

La coloration de l'échantillon a été déterminée à t = 0, après 10 jours, après 20 jours et après 30 jours.
t = 0 : 8 Apha ; t = 10 jours : 26 Apha ; t = 20 jours : 47 Apha ; t = 30 jours : 82 Apha

### Exemple 4 (comparatif)

Un échantillon d'ADAME ne comprenant pas de BHT, a été conservé dans les mêmes conditions que celles de l'exemple 3.

La coloration de l'échantillon a été déterminée à t = 0, après 10 jours, après 20 jours et après 30 jours.
t = 0 : 8 Apha ; t = 10 jours : 55 Apha
t = 20 jours : >100 Apha ; t= 30 jours : >200 Apha

### Exemple 5

On a suivi l'évolution de la coloration d'un échantillon d'ADAME maintenu à l'abri de la lumière à 30°C pendant une période allant jusqu'à 30 jours, sans ajout de BHT, et en présence de 10 ppm ou 36 ppm de BHT. Les résultats rassemblés dans le tableau 1 montrent que le BHT ajouté à une teneur de 10 ppm ne stabilise pas suffisamment l'ADAME qui présente une coloration supérieure à 100 APHA après 30 jours de stockage.

**Tableau 1**

| | | | |
|---|---|---|---|
| Teneur en BHT, ppm | 0 | 10 | 36 |
| Coloration en Apha, t=0 | 4 | 4 | 4 |
| 10 jours | 40 | 26 | 12 |
| 20 jours | 145 | 68 | 34 |
| 30 jours | >350 | 135 | 45 |

### Exemple 6 (référence)

On a reproduit l'enseignement du document EP 0850916 en mesurant la coloration (Apha) après stockage à l'abri de la lumière à 20°C pendant environ 30 jours, d'un échantillon d'ADAME (commercial) après ajout d'un composé phénolique (EMHQ, ou Topanol A) seul ou en association avec un composé phosphoré (triphényl phosphine PPh3). Les teneurs sont exprimées en ppm.

Les résultats sont rassemblés dans le tableau 2 ci-après.

**Tableau 2**

| | EMHQ | Topanol A | PPh₃ | Coloration t=0 | Coloration 9 jours | Coloration 23 jours | Coloration 28 jours |
|---|---|---|---|---|---|---|---|
| Essai 1 | 121 | | | 10 | 14 | 40 | 56 |
| Essai 2 | | 47 | | 4 | 8 | 33 | 45 |
| Essai 3 | 1042 | | | 5 | 8 | 22 | 30 |
| Essai 4 | 1014 | | 1000 | 5 | 7 | 8 | 9 |

L'ajout d'un composé phénolique substitué tel que EMHQ ou Topanol A à une teneur comprise entre 25 et 200 ppm ne permet pas de maintenir la coloration de l'ADAME après 30 jours à une valeur aussi faible que celle obtenue avec l'ajout de 46 ppm de BHT (15 Apha) selon l'exemple 1 selon l'invention.

La sélection du BHT répondant à la formule des composés phénoliques décrite dans l'art antérieur conduit donc à un effet surprenant sur la stabilisation vis-à-vis de la coloration de l'ADAME.

L'ajout d'une quantité supérieure d'EMHQ (plus de 1000 ppm) seul ne permet pas d'atteindre l'effet du BHT.

L'essai 4 confirme que la stabilisation de l'ADAME est satisfaisante en associant le dérivé phénolique EMHQ avec un composé phosphine.

L'invention, basée sur l'ajout du seul composé BHT à une teneur inférieure à 200 ppm fournit ainsi une solution plus simple et plus économique au problème de la stabilisation de l'ADAME vis-à-vis de la coloration que celle de l'art antérieur.

## Revendications

1. Procédé de fabrication en continu d'une composition d'acrylate de N,N-diméthyl aminoéthyle stabilisée vis-à-vis de la coloration, ladite composition comprenant de 25 à 200 ppm de 2,6-di-tert-butyl-4-méthyl phénol, ledit procédé comprenant une réaction de transestérification entre un acrylate léger choisi parmi l'acrylate de méthyle et l'acrylate d'éthyle, et le diméthylaminoéthanol en présence d'un catalyseur suivie d'un traitement de purification du mélange réactionnel comprenant une distillation finale de l'acrylate de N,N-diméthyl aminoéthyle purifié, **caractérisé en ce que** l'on introduit de 25 à 200 ppm de 2,6-di-tert-butyl-4-méthyl phénol à la sortie du flux gazeux distillé d'acrylate de N,N-diméthyl aminoéthyle purifié, avant condensation du produit final.

2. Procédé selon la revendication 1 comprenant les étapes suivantes :
a) on effectue une réaction de transestérification entre un acrylate léger choisi parmi l'acrylate de méthyle et l'acrylate d'éthyle, et du diméthylaminoéthanol en présence d'un catalyseur de transestérification, et en présence d'au moins un inhibiteur de polymérisation, le mélange azéotropique acrylate léger / alcool léger correspondant étant soutiré en continu pendant la réaction ;
b) on adresse le milieu brut réactionnel à une première colonne de distillation C1 sous pression réduite, dite colonne d'équeutage, le mélange brut réactionnel comprenant l'acrylate de N,N-diméthyl aminoéthyle recherché avec, comme produits légers, le diméthylaminoéthanol et l'acrylate léger n'ayant pas réagi, et, comme produits lourds, le catalyseur, le ou les inhibiteurs de polymérisation ainsi que des sous-produits lourds de réaction, permettant d'obtenir :
o en tête, un flux composé essentiellement d'acrylate de N,N-diméthyl aminoéthyle et des produits légers, comportant une fraction minoritaire de produits lourds, mais exempt ou sensiblement exempt de catalyseur ; et
o en pied, un flux composé de produits lourds avec une fraction minoritaire d'acrylate de N,N-diméthyl aminoéthyle et le catalyseur ; puis
c) on adresse le flux de tête de la première colonne de distillation C1 à une seconde colonne de distillation C2 sous pression réduite, dite colonne d'étêtage, permettant d'obtenir :
o en tête, un flux des produits légers avec une fraction minoritaire d'acrylate de N,N-diméthyl aminoéthyle ; et
o en pied, l'acrylate de N,N-diméthyl aminoéthyle contenant des traces de produits légers, des sous-produits de réaction lourds et le ou les inhibiteurs de polymérisation ; puis
d) on adresse le flux de pied de la seconde colonne de distillation C2 à une troisième colonne de distillation C3 sous pression réduite, dite colonne de rectification, dans laquelle on introduit en tête de 25 à 200 ppm de 2,6-di-tert-butyl-4-méthyl phénol, permettant d'obtenir :
o en tête, un flux gazeux d'acrylate de N,N-diméthyl aminoéthyle purifié et stabilisé vis-à-vis de la coloration qui est ensuite condensé ; et
o en pied, essentiellement le ou les inhibiteurs de polymérisation

3. Procédé selon la revendication 1 ou 2 **caractérisée** en que l'acrylate léger est l'acrylate d'éthyle.

4. Procédé selon l'une des revendications 1 à 3 dans lequel ladite composition comprend de 25 à 100 ppm de 2,6-di-tert-butyl-4-méthyl phénol.

5. Procédé selon l'une des revendications 1 à 3 dans lequel ladite composition présente une coloration inférieure à 100 Apha.

6. Procédé selon la revendication 5 dans lequel ladite composition présente une coloration inférieure à 30 Apha.

7. Procédé selon la revendication 6 dans lequel ladite composition présente une coloration inférieure à 20 Apha.

8. Procédé selon l'une des revendications 1 à 7 dans lequel ladite composition comprend en outre au moins un inhibiteur de polymérisation, choisi parmi l'hydroquinone, l'éther méthylique d'hydroquinone (EMHQ) ou leurs mélanges.

## Patentansprüche

1. Verfahren zur kontinuierlichen Herstellung einer N,N-Dimethylaminoethylacrylatzusammensetzung, die gegenüber Verfärbung stabilisiert ist, wobei die Zusammensetzung 25 bis 200 ppm 2,6-Di-tert-butyl-4-methylphenol umfasst, wobei das Verfahren eine Umesterungsreaktion zwischen einem leichten Acrylat, das aus Methylacrylat und Ethylacrylat ausgewählt wird, und Dimethylaminoethanol in Gegenwart eines Katalysators gefolgt von einer Reinigungsbehandlung der Reaktionsmischung, die eine abschließende Destillation des gereinigten N,N-Dimethylaminoethylacrylats umfasst, umfasst, **dadurch gekennzeichnet, dass** man am Ausgang des destillierten Abgasstroms von gereinigtem N,N-Dimethylaminoethylacrylat vor der Kondensation des Endprodukts 25 bis 200 ppm 2,6-Di-tert-butyl-4-methylphenol einträgt.

2. Verfahren nach Anspruch 1, das die folgenden Schritte umfasst:
a) man führt eine Umesterungsreaktion zwischen einem leichten Acrylat, das aus Methylacrylat und Ethylacrylat ausgewählt wird, und Dimethylaminoethanol in Gegenwart eines Umesterungskatalysators und in Gegenwart mindestens eines Polymerisationsinhibitors durch, wobei das entsprechende azeotrope Gemisch von leichtem Acrylat/leichtem Alkohol während der Reaktion kontinuierlich abgezogen wird;
b) man führt das rohe Reaktionsmedium einer ersten Niederdruck-Destillationskolonne C1, die als Tailing-Säule bezeichnet wird, zu, wobei die rohe Reaktionsmischung das gewünschte N,N-Dimethylaminoethylacrylat mit Dimethylaminoethanol und nicht umgesetztem leichtem Acrylat als leichten Produkten und dem Katalysator, dem Polymerisationsinhibitor bzw. den Polymerisationsinhibitoren sowie schweren Reaktionsnebenprodukten als schweren Produkten umfasst, wodurch Folgendes erhalten werden kann:
o am Kopf ein Strom, der im Wesentlichen aus N,N-Dimethylaminoethylacrylat und leichten Produkten besteht und eine untergeordnete Fraktion von schweren Produkten enthält, aber frei oder weitgehend frei von Katalysator ist; und
o am Sumpf ein Strom, der aus schweren Produkten mit einer untergeordneten Fraktion von N,N-Dimethylaminoethylacrylat und dem Katalysator besteht; dann
c) man führt den Kopfstrom der ersten Destillationskolonne C1 einer zweiten Niederdruck-Destillationskolonne C2, die als Topping-Säule bezeichnet wird, zu, wodurch Folgendes erhalten werden kann:
o am Kopf ein Strom von leichten Produkten mit einer untergeordneten Fraktion von N,N-Dimethylaminoethylacrylat; und
o am Sumpf N,N-Dimethylaminoethylacrylat, das Spuren von leichten Produkten, schweren Reaktionsnebenprodukten und dem Polymerisationsinhibitor bzw. den Polymerisationsinhibitoren enthält; dann
d) man führt den Sumpfstrom der zweiten Destillationskolonne C2 einer dritten Niederdruck-Destillationskolonne C3, die als Rektifikationskolonne bezeichnet wird, zu, in die man am Kopf 25 bis 200 ppm 2,6-Di-tert-butyl-4-methylphenol einträgt, wodurch Folgendes erhalten werden kann:
o am Kopf ein gasförmiger Strom von gereinigtem und gegenüber Verfärbung stabilisiertem N,N-Dimethylaminoethylacrylat, der anschließend kondensiert wird; und
o am Sumpf im Wesentlichen der Polymerisationsinhibitor bzw. die Polymerisationsinhibitoren.

3. Verfahren nach Anspruch 1 oder 2, **dadurch gekennzeichnet, dass** es sich bei dem leichten Acrylat um Ethylacrylat handelt.

4. Verfahren nach einem der Ansprüche 1 bis 3, wobei die Zusammensetzung 25 bis 100 ppm 2,6-Di-tert-butyl-4-methylphenol umfasst.

5. Verfahren nach einem der Ansprüche 1 bis 3, wobei die Zusammensetzung eine Verfärbung von weniger als 100 Apha aufweist.

6. Verfahren nach Anspruch 5, wobei die Zusammensetzung eine Verfärbung von weniger als 30 Apha aufweist.

7. Verfahren nach Anspruch 6, wobei die Zusammensetzung eine Verfärbung von weniger als 20 Apha aufweist.

8. Verfahren nach einem der Ansprüche 1 bis 7, wobei die Zusammensetzung außerdem mindestens einen Polymerisationsinhibitor umfasst, der aus Hydrochinon, Hydrochinonmethylether (HQME) oder Mischungen davon ausgewählt ist.

## Claims

1. A process for the continuous production of a composition of N,N-dimethylaminoethyl acrylate that is stabilized with respect to coloration, said composition comprising from 25 to 200 ppm of 2,6-di-tert-butyl-4-methylphenol, said process comprising a transesterification reaction between a light acrylate selected from methyl acrylate and ethyl acrylate, and dimethylaminoethanol in the presence of a catalyst, followed by a treatment for purification of the reaction mixture comprising a final distillation of the purified N,N-dimethylaminoethyl acrylate, **characterized in that** 25 to 200 ppm of 2,6-di-tert-butyl-4-methylphenol are introduced at the outlet of the distilled gaseous stream of purified N,N-dimethylaminoethyl acrylate, before condensation of the final product.

2. The process as claimed in claim 1, comprising the following steps:
a) a transesterification reaction is carried out between a light acrylate selected from methyl acrylate and ethyl acrylate, and dimethylaminoethanol in the presence of a transesterification catalyst and in the presence of at least one polymerization inhibitor, the corresponding azeotropic mixture of light acrylate/light alcohol being drawn off continuously during the reaction;
b) the crude reaction mixture is conveyed to a first distillation column C1, referred to as tailing column, under reduced pressure, the crude reaction mixture comprising the desired N,N-dimethylaminoethyl acrylate with, as light products, dimethylaminoethanol and unreacted light acrylate and, as heavy products, the catalyst, the polymerization inhibitor(s) and also heavy reaction by-products, making it possible to obtain:
o at the top, a stream composed essentially of N,N-dimethylaminoethyl acrylate and light products, comprising a minor fraction of heavy products but devoid, or substantially devoid, of catalyst; and
o at the bottom, a stream composed of heavy products with a minor fraction of N,N-dimethylaminoethyl acrylate and the catalyst; then
c) the top stream from the first distillation column C1 is conveyed to a second distillation column C2, referred to as topping column, under reduced pressure, making it possible to obtain:
o at the top, a stream composed of light products with a minor fraction of N,N-dimethylaminoethyl acrylate; and
o at the bottom, N,N-dimethylaminoethyl acrylate containing traces of light products, heavy reaction by-products and the polymerization inhibitor(s); then
d) the bottom stream from the second distillation column C2 is conveyed to a third distillation column C3, referred to as rectification column, under reduced pressure, into the top of which 25 to 200 ppm of 2,6-di-tert-butyl-4-methylphenol are introduced, making it possible to obtain:
o at the top, a gaseous stream of purified N,N-dimethylaminoethyl acrylate that is stabilized with respect to coloration which is subsequently condensed; and
o at the bottom, essentially the polymerization inhibitor(s).

3. The process as claimed in claim 1 or 2, **characterized in that** the light acrylate is ethyl acrylate.

4. The process as claimed in any one of claims 1 to 3, wherein said composition comprises from25 to 100 ppm, of 2,6-di-tert-butyl-4-methylphenol.

5. The process as claimed in any one of claims 1 to 3, wherein said composition has a coloration of less than 100 Apha.

6. The process of claim 5, wherein said composition has a coloration of less than 30 Apha,

7. The process of claim 6, wherein said composition has a coloration of less than 20 Apha,

8. The process as claimed in any one of claims 1 to 7, wherein said composition comprises at least one polymerization inhibitor, selected from hydroquinone, hydroquinone methyl ether (MEHQ) or mixtures thereof.
